# EUROPEAN PATENT APPLICATION

(11) **EP 4 497 404 A1**
(43) Date of publication of application: **29.01.2025**
(21) Application number: 24190193.3
(22) Date of filing: 23.07.2024
(51) Int. Cl.: A61B 18/14, A61B 5/00

(54) **CATHETER END EFFECTOR WITH NONPLANAR SUBSTRATE**

(30) Priority: 24.07.2023 US 202318357828
(71) Applicant: Biosense Webster (Israel) Ltd., 2066717 Yokneam (IL)
(72) Inventor: EBRAHIMI, Babak, Irvine, 92618 (US); RODRIGUEZ SOTO, Juan, Irvine, 92618 (US); VAN NIEKERK, Pieter Emmelius, Irvine, 92618 (US); ABBAS, Mohammad, Irvine, 92618 (US)
(74) Representative: Carpmaels & Ransford LLP

(57) **Abstract**

An end effector for a catheter. The end effector can include a substrate extending along a longitudinal axis from a proximal portion of the end effector to a distal portion of the end effector. The substrate can include a longitudinal plane, the longitudinal axis running through the longitudinal plane, a first face disposed on a first side of the substrate and oblique to the longitudinal plane; a second face, opposite the first face, disposed on a second side of the substrate; a first group of electrodes disposed on the first face; and a second group of electrodes disposed on the second face.

## Description

### FIELD

The present invention relates generally to minimally invasive medical devices, and in particular sensing catheters, and further relates to, but not exclusively, cardiac mapping catheters.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for the end effector of a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. If the internal members are too stiff, manipulation of the catheter in the body organ can be difficult and can prevent electrodes from contacting the tissue.

### SUMMARY

There is provided, in accordance with an example of the present invention, an end effector for a catheter. The end effector can include a substrate extending along a longitudinal axis from a proximal portion of the end effector to a distal portion of the end effector. The substrate can include a longitudinal plane with the longitudinal axis running through the longitudinal plane. A first face can be disposed on a first side of the substrate and be oblique to the longitudinal plane and a second face, opposite the first face, can be disposed on a second side of the substrate. A first group of electrodes can be disposed on the first face and a second group of electrodes can be disposed on the second face.

The disclosed technology includes a nonplanar end effector for a mapping catheter. The end effector can include a sloped substrate extending along a longitudinal plane extending along a longitudinal axis from a proximal end of the end effector to a distal end of the end effector. The substrate can thin progressively in a direction orthogonal to the longitudinal plane. Nonplanar end effector can include a first plurality of electrodes disposed on a first face of the sloped substrate and a second plurality of electrodes disposed on a second face of the sloped substrate.

The disclosed technology includes a system for performing a medical procedure. The system can include an elongate shaft extending along a longitudinal axis from a proximal end to a distal end, an end effector disposed on the distal end of the elongate shaft. The end effector can include a substrate extending along a longitudinal axis from a proximal portion of the end effector to a distal portion of the end effector. The substrate can include a first face disposed on a first side of a longitudinal plane and oblique thereto, a second face disposed on a second side of the longitudinal plane and oblique thereto, a first group of electrodes disposed on the first face, and a second group of electrodes disposed on the second face.

The disclosed technology includes a method of providing a stiffened flexible end effector. The method can include forming a substrate along a longitudinal plane extending along a longitudinal axis from a proximal end to a distal end, forming a first face on a first side of the substrate, forming a second face on a second side, opposite the first side, of the substrate, and sloping the first face toward the longitudinal plane as the first face extends from the proximal end of the substrate to the distal end of the substrate. The method can include forming a first group electrodes along the first face and forming a second group of electrodes along the second face.

### BRIEF DESCRIPTION OF THE DRAWINGS

FIG. 1 is a schematic pictorial illustration of a medical system including a catheter with an end effector having a nonplanar substrate, in accordance with an example of the present invention;
FIG. 2 is an illustration of a top view of a system for a medical procedure, in accordance with an example of the present invention;
FIG. 3A is an illustration of a cross-section an end effector, in accordance with an example of the present invention;
FIG. 3B illustrates a detailed view of the end effector of FIG. 3A;
FIG. 4A is an illustration of a cross-section of an end effector, in accordance with an example of the present invention;
FIG. 4B illustrates a detailed view of the distal portion of the end effector of FIG. 4A;
FIG. 4C illustrates a detailed view of the proximal portion of the end effector of FIG. 4A;
FIG. 5A is an illustration of a top view of an end effector, in accordance with an example of the present invention;
FIG. 5B is an illustration of a bottom view of an end effector, in accordance with an example of the present invention;
FIG. 6 is an illustration of an end effector encapsulated in a flexible material, in accordance with an example of the present invention;
FIG. 7A is a flowchart of a method of providing a stiffened flexible end effector, in accordance with an example of the present invention;
FIG. 7B is a flowchart of a method of providing a stiffened flexible end effector, in accordance with an example of the present invention; and
FIG. 7C is a flowchart of a method of providing a stiffened flexible end effector, in accordance with an example of the present invention.

### DETAILED DESCRIPTION

The disclosed technology relates to improving the performance of end effectors, for example those used with cardiac mapping catheters. The systems, devices, and methods of the disclosed technology enhance various aspects of mapping and ablation catheter performance, including, but not limited to: mapping resolution, electrode contact with the target anatomy, delivery of the end effector to the target anatomy, biocompatibility, end effector stiffness, and atraumaticity. Various aspects of the present disclosure enhance the ability of the end effector to conform to target tissue by modulating the stiffness along the length of the end effector and the torsional stiffness (rotation about the longitudinal axis) of the end effector. The sloping nature of the substrate of the end effector generally lends itself to decreasing stiffness along the length of the end effector and the torsional stiffness. Other aspects and/or components of the end effectors, such as electrode height and angle, the angle at which the end effector slopes, and the addition of slots, a framework, and a flexible encapsulating material can be used to tune the ability of the end effector's conformation to a tissue surface.

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several examples, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred example. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), sometimes referred to as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE is sometimes referred to interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

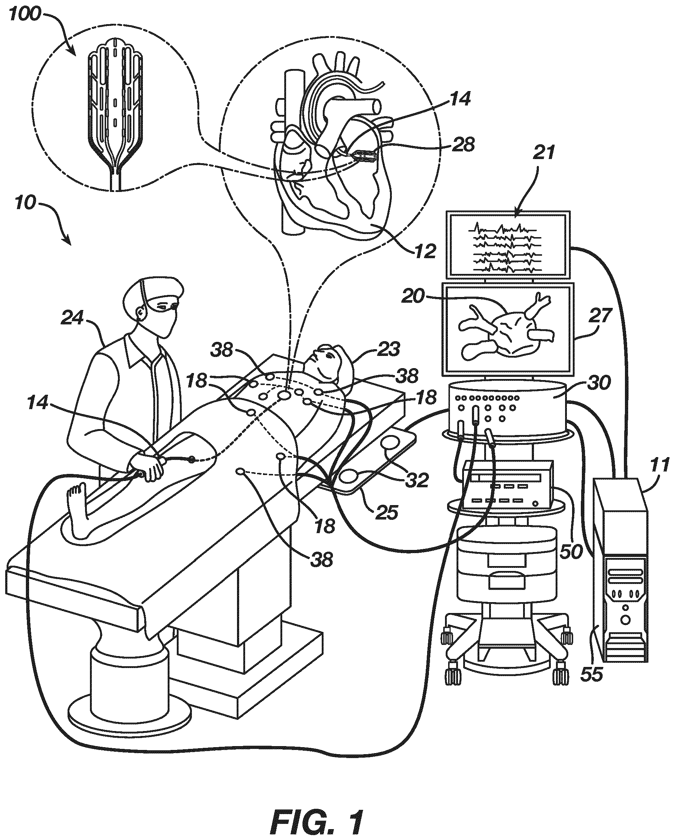

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., end effector 100) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes optionally distributed over end effector 100 and configured to sense the IEGM signals as described herein. Catheter 14 may additionally include a position sensor embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix hereto.

System 10 includes one or more electrode patches 38 positioned for skin contact on patient 23 to establish location reference for location pad 25 as well as impedance-based tracking of electrodes 26. For impedance-based tracking, electrical current is directed toward electrodes and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix hereto.

A recorder 11 displays electrograms 21 captured with body surface ECG electrodes 18 and intracardiac electrograms (IEGM) captured with electrodes of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

In order to achieve the desired stiffness, the mapping resolution, electrode contact with target anatomy, and conformity of the end effector disclosed herein to flat, curved, irregular and/or nonplanar tissue surfaces found in the target anatomy, the end effector has a sloped substrate, among other components.

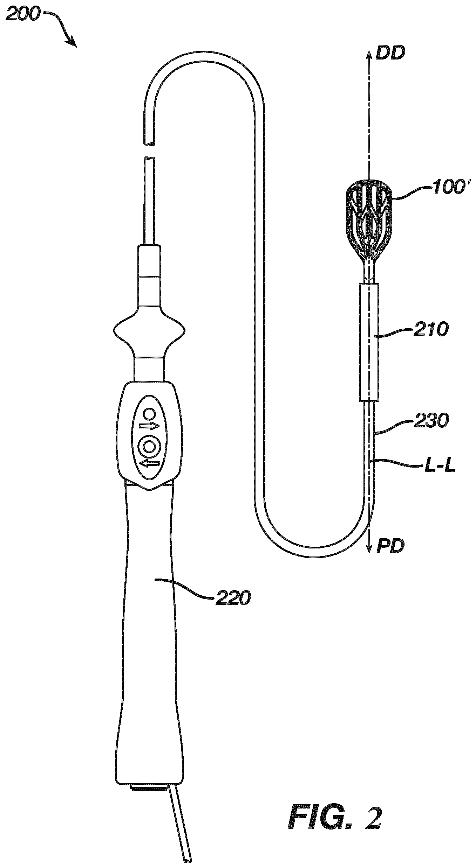

The disclosed technology includes a system 200 for performing a medical procedure as shown in FIG. 2. The system 200 can include an elongate shaft 230 extending along a longitudinal axis L-L from a proximal end 234 to a distal end 232, an end effector 100 disposed on the distal end 232 of the elongate shaft 230. The elongate shaft 230 can be configured to deliver the end effector 100' out of a sheath 210. For simplicity, only a portion of the sheath 210 is shown in FIG. 2. Note that end effector 100' has an alternative configuration to end effector 100 depending on the use case but the disclosure provided herein is applicable to both embodiments.

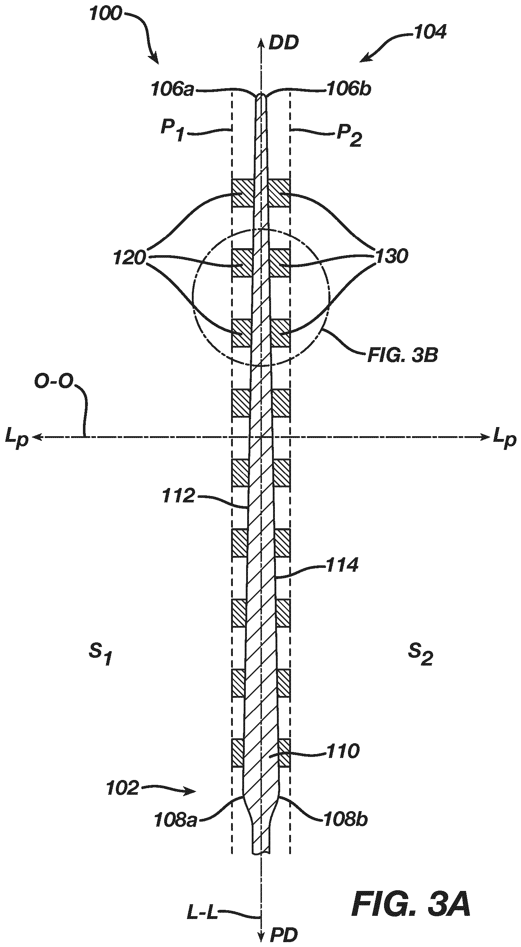

A physician can manipulate the elongate shaft 230 and the end effector 100' with handle 220. Appropriate examples for system 200 and its subcomponents such as handle 220, sheath 210, elongate shaft 230, and others not mentioned herein are described in US Patent publication No. 2021/0369339, which is incorporated herein by reference and included in the Appendix attached hereto. System 200 can further include a pull wire (not shown) extending through the elongate shaft 230 and including a distal end (not shown) coupled to a proximal portion 102 (shown in FIG. 3A) of the end effector 100 or 100'. Details of the manipulation of end effectors via pull wires are described in US Patent Publication No. 2021/0369339, which is incorporated herein by reference and included in the Appendix attached hereto.

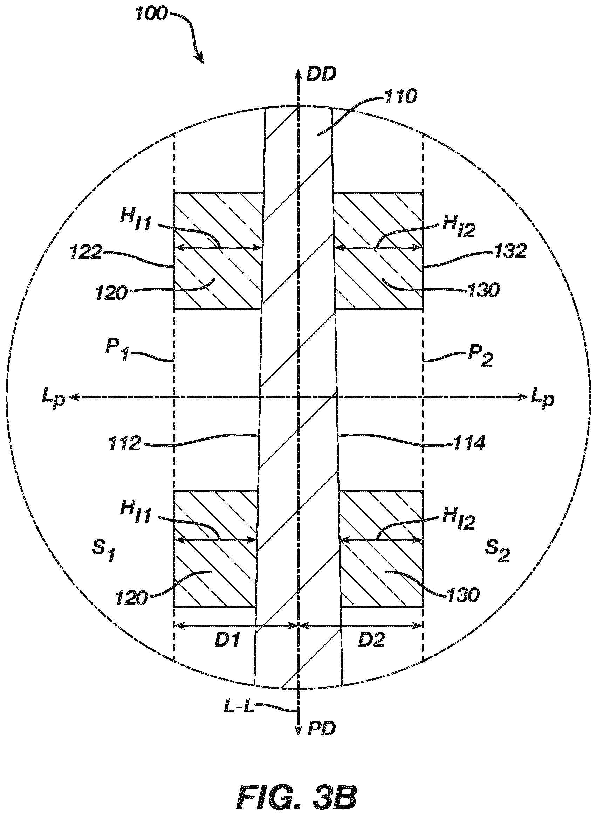

FIG. 3A shows end effector 100 or 100' in greater detail in a side cross-sectional view. The end effector 100 can include a substrate 110 extending along a longitudinal axis L-L from the proximal portion 102 of the end effector 100 or 100' to a distal portion 104 of the end effector 100. The substrate 110 can include a longitudinal plane Lₚ with the longitudinal axis L-L running through the longitudinal plane Lₚ. In other words, the longitudinal plane Lₚ can extend along the longitudinal axis and in a direction in and out of the page when viewing FIG. 3A, effectively bisecting the end effector 100, with approximately half of the end effector 100 lying on one side of the longitudinal plane Lₚ and the other half lying on the other side of the longitudinal plane Lₚ. A first face 112 can be disposed on a first side Si of the substrate 110. The first face 112 can extend in a direction that is oblique to the longitudinal plane Lₚ (i.e., the first face 112 can extend in a direction that is not parallel nor at a right angle to the longitudinal plane Lₚ). In other words, end effector 100, namely the substrate 110, can be thicker at the proximal portion 102 and narrower toward the distal end 104 which has the effect of progressively reducing the stiffness of end effector from the proximal portion 102 to the distal portion 104. As discussed in more detail in relation to FIG. 3B below, the electrodes 120, 130 can vary in thickness as well.

Stated otherwise, a distance between the first face 112 and the longitudinal plane Lₚ at a proximal end can be greater than a distance between the first face 112 and the longitudinal plane Lₚ at a distal end. A second face 114, opposite the first face 112, can be disposed on a second side S₂ of the substrate 110. The end effector can further include a first group of electrodes 120 disposed on the first face 112 and a second group of electrodes 130 disposed on the second face 114.

Though the term "face" is used for both first face 112 and second face 114 and how they relate to longitudinal plane Lₚ, it is to be understood that first face 112 and second face 114 are not necessarily completely planar. First face 112 and second face 114 can have undulating profiles, through holes, scallops, and the like, all of which can be included for the purpose of tuning the stiffness and/or the conformability to the tissue of the end effector 100. Generally, end effector 100 is roughly spatula shaped. The end effector 100 can further include a substrate having a hollow profile. Furthermore, the end effector 100 can include inserts or frame members made from a composite matrix of materials (metals, polymers, ceramics, etc.) stacked in various orientations (longitudinal, perpendicular, axial, etc.) which can form an array. Such inserts or frame members can be woven, spun, braided, molded, casted, printed (including three-dimensional printing) or manufacturing using any other suitable manufacturing process.

In some examples, the second face 114 can be oblique to the longitudinal plane Lₚ. That is to say, the second face 114 can be nonparallel to the longitudinal plane Lₚ and the first face 112. The second face 114 can lie at an angle relative to the longitudinal plane Lₚ approximately equal and opposite to the angle at which the first face 112 lies relative to the longitudinal plane Lₚ, such that the second face 114 is a mirror image of the first face 112 taken across the longitudinal plane Lₚ. Stated otherwise, a distance between the second face 114 and the longitudinal plane Lₚ at a proximal end can be greater than a distance between the second face 114 and the longitudinal plane Lₚ at a distal end.
Alternatively, the angle at which the first face 112 lies relative to the longitudinal plane Lₚ can be different than the angle at which the first face 112 lies relative to the longitudinal plane Lₚ. In other examples, only the first face 112 or the second face 114 can extend in a direction that is oblique to the longitudinal plane Lₚ while the other of the first face 112 or the second face 114 extends in a direction that is approximately parallel to the longitudinal plane Lₚ.

The end effector 100 can further define a first offset plane P₁ offset from the first face 112 and parallel to the longitudinal plane Lₚ and a second offset plane P₂ offset from second face 114 and parallel to the longitudinal plane Lₚ. In some examples, the first group of electrodes 120 can extend outwardly from the first face 112 to the first offset plane P₁ and the second group of electrodes 130 can extend outwardly from the second face 114 to the second offset plane P₂. In other words, the first group of electrodes 120 can be projected from the first face 112 such that, if end effector 100 is rested on a flat surface, the longitudinal plane Lₚ lies parallel to the flat surface. Alternatively, the plane P₁ can be offset from the first face 112 and oblique to the longitudinal plane Lₚ such that some or all of the first group of electrodes 120 can be projected from the first face 112 and, if end effector 100 is rested on a flat surface, the first face 112 lies generally parallel to the flat surface. Either of these configurations for the first group of electrodes 120 can be combined with similar configurations of the second group of electrodes 130.

Stated otherwise, the disclosed technology includes a nonplanar end effector 100 for a mapping or ablation catheter. Though end effector 100 and substrate 110 may be described as nonplanar, some subcomponents can lie in a flat plane. For example, as discussed in more detail below, contact surfaces 122 of the first group of electrodes 120 can lie in a flat plane, and a non-conductive flexible material 150 may result in an end effector 100 which as an overall flat outer profile. The term "nonplanar" as used in relation to the end effector 100 is not meant to exclude these examples. The end effector 100 can include a sloped substrate 110 extending along a longitudinal plane Lₚ extending along a longitudinal axis L-L from a proximal end 102 of the end effector 100 to a distal end 104 of the end effector 100. The substrate 110 can thin progressively in a direction orthogonal O-O to the longitudinal plane Lₚ. Nonplanar end effector 100 can include a first plurality of electrodes 120 disposed on a first face 112 of the sloped substrate 110 and a second plurality of electrodes 130 disposed on a second face 114 of the sloped substrate 110. In some examples, the sloped substrate 110 can be a flexible circuit (flex circuit). In some examples, flexible circuit can be made primarily of polyimide. In other examples, it can be made of any of biocompatible polyimides, glass-reinforced epoxy laminate materials, copper, or graphene, alone or in combination with other biocompatible materials or with each other. The flexible circuit can include conductive traces connecting the first 120 and second 130 groups of electrodes to components of system 10, for at least the purpose of acquiring signals and delivering energy.

As shown in FIG. 3B, the first group of electrodes 120 can each include an individual height H_{I1} from the first face 112 to the first offset plane P₁. In some examples, the second group of electrodes 130 can each include an individual height H_{I2} from the second face 114 to the second offset plane P₂. As seen in FIG. 3B, the individual height Hn of the more distal first electrode 120 is taller than the individual height H_{I1} of the more proximal first electrode 120. Similarly, the individual height H_{I2} of the more distal second electrode 130 is taller than the individual height H_{I2} of the more proximal second electrode 130. In some examples, such as that shown in FIG. 3A, this trend can be true for the entire end effector 100, meaning that individual heights H_{I1} and H_{I2} of each electrode 120, 130 is greater than that of electrodes 120, 130 positioned more proximally i.e. closer to the proximal end 102 of the end effector 100. In some examples, the individual height Hn of each of the first electrodes can be equal to the individual height H_{I2} of a respective second electrode 130 positioned similarly in relation to the longitudinal axis, or, in other words, a second electrode 130 which is an equal distance from the proximal end 102 of the end effector 100. The first offset plane P₁ can be parallel to the longitudinal plane Lₚ and separated therefrom by a first distance D₁. The second offset plane P₂ can be parallel to the longitudinal plane Lₚ and separated therefrom by a second distance D₂. In some examples, the second distance D₂ is equal to the first distance D₁ while in other examples the second distance D₂ is different than the first distance D₁. The first plurality of electrodes 120 can include a respective plurality of first contact surfaces 122. The second plurality of electrodes 130 can include a respective plurality of second contact surfaces 132. The plurality of first contact surfaces 122 and the plurality of second contact surfaces 132 can be parallel to the longitudinal plane Lₚ as shown in FIG. 3B. Contact surfaces 122 are the surfaces of electrodes 120 which lie in the first offset plane P₁ and are configured to contact tissue targeted for sensing and/or ablation. The term "contact" as used herein can refer to the component touching tissue or can refer to electrical contact. For example, contact surfaces 122 can be coated or otherwise prevented from touching tissue but may still be capable of delivering (e.g., ablation of tissue) or receiving electrical current (e.g., acquisition of physiological signals, such as the propagation of electrical currents through cardiac tissue) passing through the tissue.

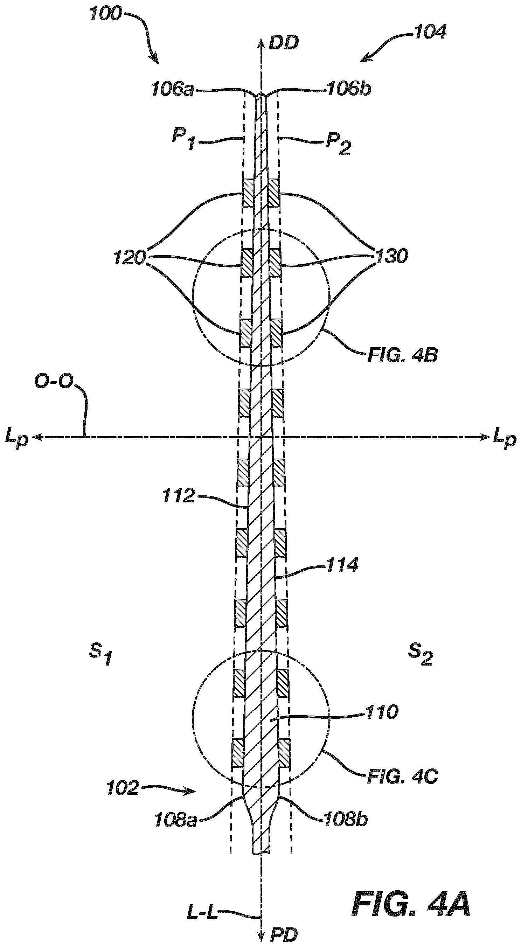

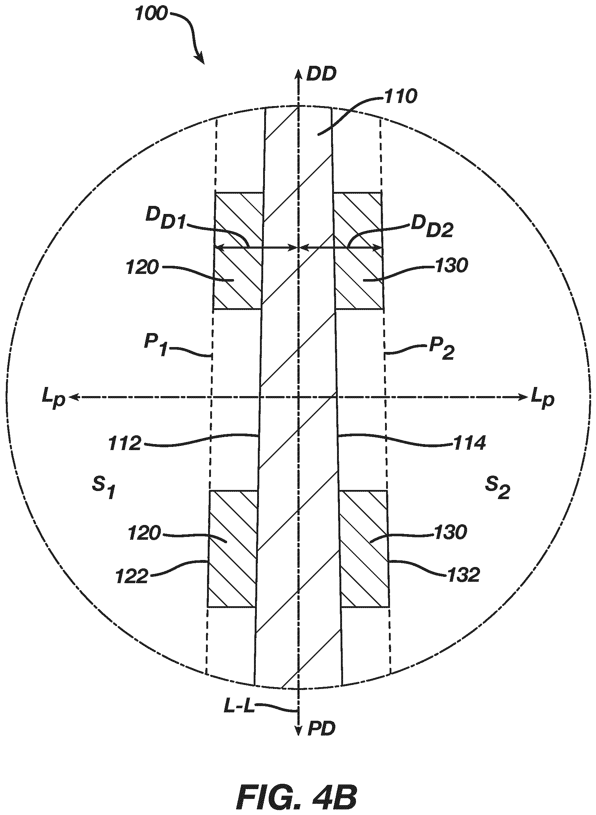

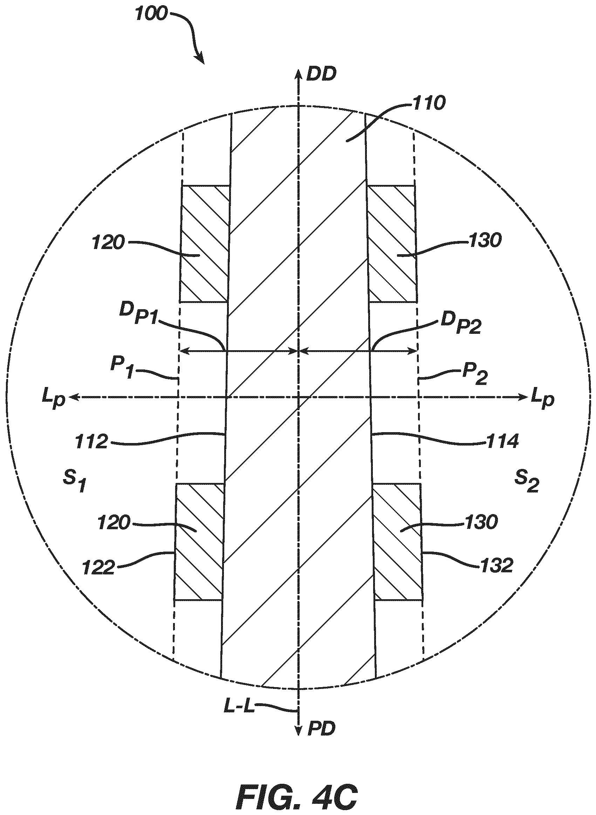

In another example, shown in FIG. 4A, end effector 100 is symmetrical across longitudinal plane Lₚ and the electrodes 120, 130 are flat against the first face 112 and the second face 114. As such, first electrodes 120 and second electrodes 130 extend up to the first offset plane P₁ and the second offset plane P₂, respectively, each having the same height. In other words, distal end 106a of the first face 112 is separated from the first offset plane P₁ by a first distal distance D_{D1} (as shown in FIG. 4B), a proximal end 108a of the first face 112 is separated from the first offset plane P₁ by a first proximal distance D_{P1} (as shown in FIG. 4C), and the first distal distance D_{D1} does not equal the first proximal distance D_{P1}. That is, the first proximal distance D_{P1} can be greater than the first distal distance D_{D1}. In some examples, this results in the first group of electrodes 120 having a uniform height and lying flatly against and parallel to the first face 112.

A distal end 106b of the second face 114, in some examples, is separated from the second offset plane P₂ by a second distal distance D_{D2} (as shown in FIG. 4B), a proximal end 108b of the second face 114 is separated from the second offset plane P₂ by a second proximal distance D_{P2} (as shown in FIG. 4C), and the second distal distance D_{D2} does not equal the second proximal distance D_{P2}. That is, the second proximal distance D_{P2} can be greater than the second distal distance D_{D2}. The second group of electrodes 130 can have a uniform height and lie flatly against and parallel to the second face 114. As seen in FIG. 4B, the plurality of first contact surfaces 122 can be parallel to the first face 112 and the plurality of second contact surfaces 132 can be parallel to the second face 114. Again, this can in some examples result in end effector 100 being narrower at the distal end 104 than at the proximal end 102, thus having gradually decreasing stiffness in the distal direction DD. This narrowing can increase the ability of end effector 100 to conform to tissue surfaces, thus allowing electrodes 120, 130 to better contact said tissue and more effectively acquire signals and/or deliver ablative energy.

In some examples, for example when the end effector 100 is symmetrical across the longitudinal plane Lₚ, the first proximal distance D_{P1} is equal to the second proximal distance D_{P2}, and wherein the first distal distance D_{D1} is equal to the second distal distance D_{D2}. Thus, there is an equal amount of substrate 110 lying on side Si and side S₂ of the longitudinal plane Lₚ.

In some examples, the first group of electrodes 120 can include at least one first mapping electrode and/or at least one first ablation electrode. The second group of electrodes 130 can include at least one second mapping electrode and/or at least one second ablation electrode. Electrodes 120 and 130 can be configured to detect electrophysiological signals or transmit ablative energy (AC or DC) from an energy generator to the tissue according to the various ablation methods previously described (e.g., RF, IRE, etc.). The cross-sectional profiles as shown in FIGs. 3A-4C and the nonplanar nature of end effector 100 improve the ability of the end effector 100 (and thus electrodes 120, 130) to conform to tissue, acquire signals, and deliver energy.

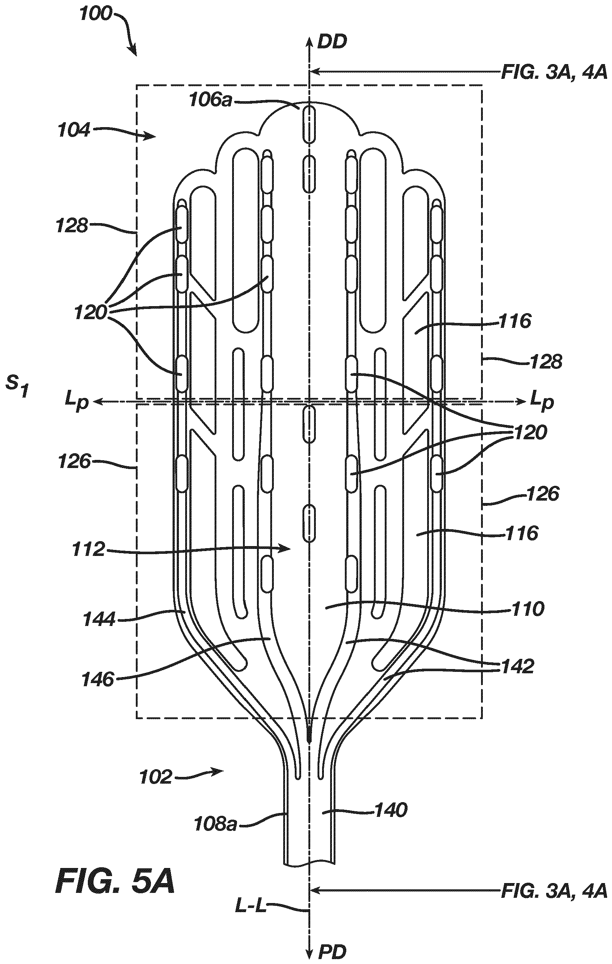

FIG. 5A illustrates a view of the first side 112 of the end effector. As shown, the first group of electrodes 120 can be disposed on the first face 112 such that a distal subset 128 of the first group of electrodes 120 is nearer the distal portion 104 than a proximal subset 126 of the first group of electrodes 120. The distal subset 128 of the first group of electrodes 120 can include approximately twice as many electrodes as the proximal subset 126 of the first group of electrodes 120. Generally, there can be more first electrodes 120 in the distal subset 128 than in the proximal subset 126.

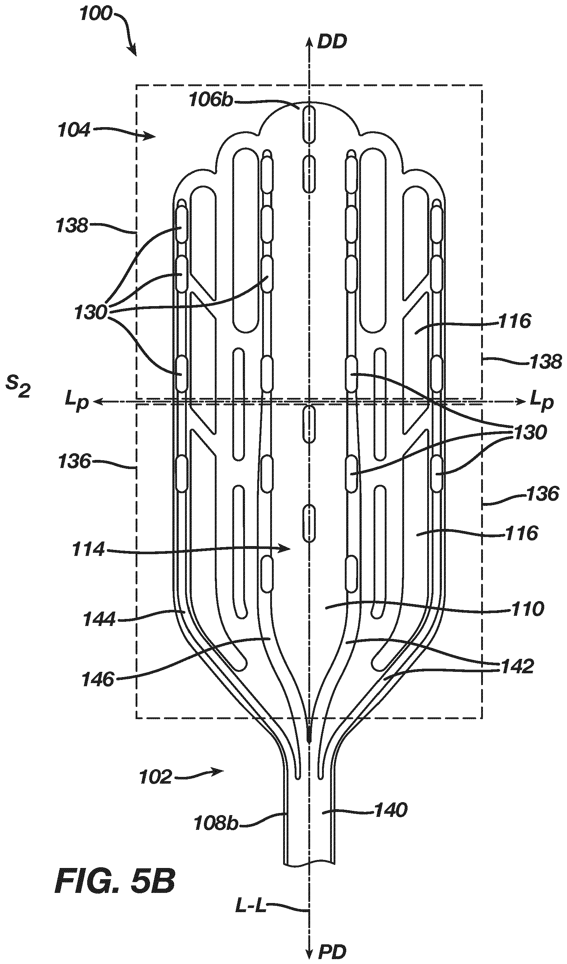

The same can be true for the second face 114 and the distribution of the second group of electrodes 130. As shown in FIG. 5B, the second group of electrodes 130 can be disposed on the second face 114 such that a distal subset 138 of the second group of electrodes 130 is nearer the distal portion 104 than a proximal subset 136 of the second group of electrodes 130 with the distal subset 138 of the second group of electrodes 130 including approximately twice as many electrodes as the proximal subset 136 of the second group of electrodes 130.

Referring back to FIGs. 3A - 4C, at least a portion of the first group of electrodes 120 can be aligned with at least a portion of the second group of electrodes 130 in a direction O-O orthogonal to the longitudinal plane Lₚ to form respective aligned pairs of first 120 and second 130 electrodes.

Aligned pairs of electrodes can be aligned generally transverse to the longitudinal axis L-L. Pairs of opposite facing electrodes can be utilized to cancel noise, with one electrode sensing tissue directly and the opposite electrode sensing far-field signals which typically may include noise, as is appreciated by those skilled in the pertinent art.

FIGs. 5A-5B show a framework 140 lying in the substrate 110 and coplanar with the longitudinal plane Lₚ. The framework 140 can include a plurality of tines 142 joined proximate a proximal end 108 of the end effector 100 and extending distally therefrom. The plurality of tines 142 can include two outer tines 144 and two inner tines 146, with an outer tine 144 and an inner tine 146 disposed on either side of the longitudinal axis L-L. In some examples, the two inner tines 146 narrow progressively in a distal direction DD. The narrowing of the tines 142 can be configured to modulate the bending stiffness of the end effector 100. The aligned pairs of first and second electrodes can be aligned with the framework 140 in the orthogonal direction O-O. In some examples, the substrate 110 can further define a plurality of elongate slots 116 lying generally parallel to the longitudinal axis L-L and disposed between each outer tine 144 and inner tine 146. Elongate slots 116 can be shaped to modify the bending stiffness and the torsional stiffness (rotation about longitudinal axis L-L) of the end effector 100 to enable it to conform to a tissue surface.

Framework 140 can be thought of as a forked plurality of stiffness increasing members serving to provide support to end effector 100.

The framework 140 can be formed from a planar or cylindrical stock of material using any suitable method. For example, the framework 140 can be formed by cutting, laser cutting, stamping, etc. In some examples, the substrate 110 can include a flexible circuit. In some examples, the substrate 110 can include a framework, such as framework 140 described above. The framework 140 can include nitinol.

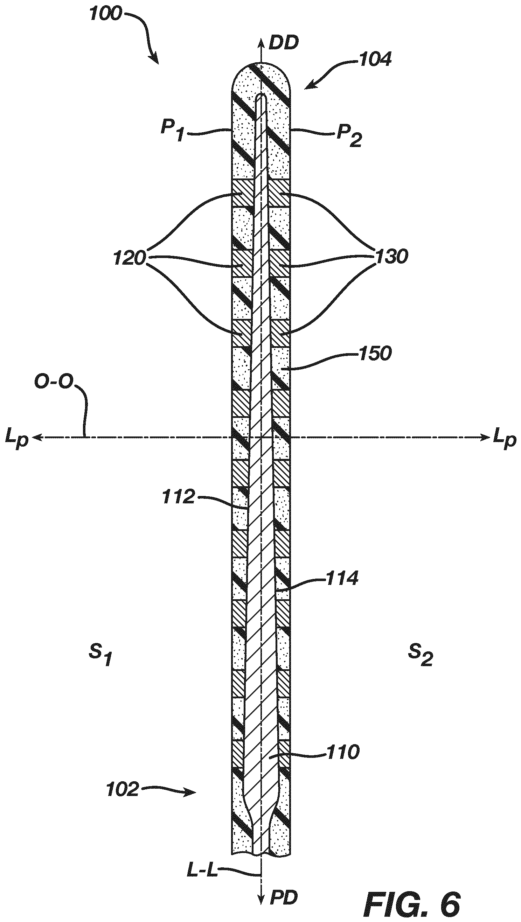

As shown in FIG. 6, end effector 100 can be at least partially encapsulated by a non-conductive flexible material 150. This nonconductive flexible material 150 can serve at least in part to enhance the atraumaticity of the end effector 100 and to protect the subject from sharp edges. In some examples, the flexible non-conductive material 150 can include biocompatible silicone. In other examples, the flexible non-conductive material 150 can include biocompatible flexible polymers, thermoelastic polymer materials, and dielectric materials. The flexible non-conductive material 150 can optionally be colored to facilitate laser cutting. In some examples, the framework 140 can be affixed to the substrate 110 by non-conductive flexible encapsulating material 150. Contact surfaces 122 and 132 can be exposed to the ambient environment through the non-conductive flexible material 150.

Furthermore, the non-conductive flexible material 150 can be thickened at various points in the longitudinal direction L-L and the orthogonal direction O-O to have a profile that compliments the variable stiffness of the substrate 110. For example, the framework 150 can be shaped such that it contributes more to the overall stiffness of the end effector 100 in the torsional stiffness (rotation about the longitudinal axis L-L) than to the longitudinal direction L-L. Alternatively, or additionally, the flexible material 150 can be formed such that it encourages folding/creasing of the end effector between columns, rows, and/or portions (for example portions 126, 128, 136, and 138 shown in FIGs. 5A and 5B) or electrodes. Flexible non-conductive material 150 can also form a blunt distal end 104.

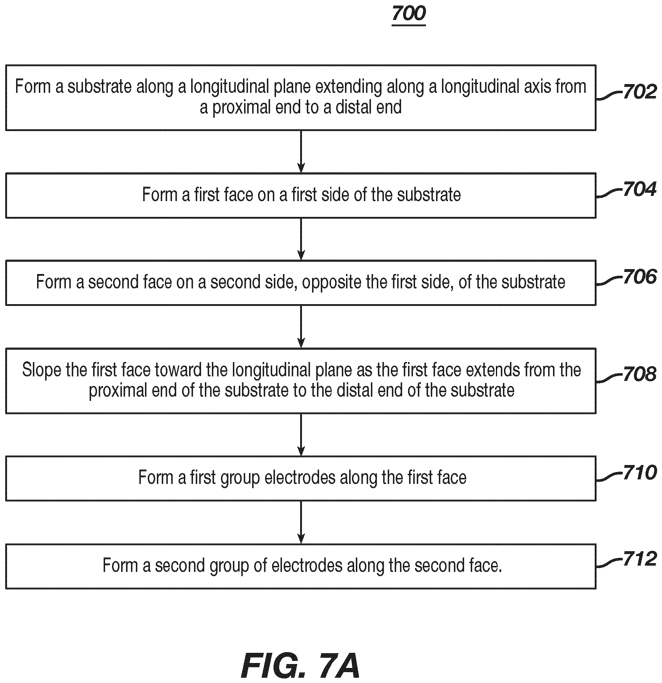

FIGs. 7A-7C shows a method 700 of providing a stiffened flexible end effector. Method 700 can in some examples provide end effector 100 as detailed above, with all of its components and subcomponents. As shown in FIG. 7A, method 700 can include forming 702 a substrate along a longitudinal plane extending along a longitudinal axis from a proximal end to a distal end, forming 704 a first face on a first side of the substrate, forming 706 a second face on a second side, opposite the first side, of the substrate, sloping 708 the first face toward the longitudinal plane as the first face extends from the proximal end of the substrate to the distal end of the substrate, forming 710 a first group electrodes along the first face, and forming 712 a second group of electrodes along the second face.

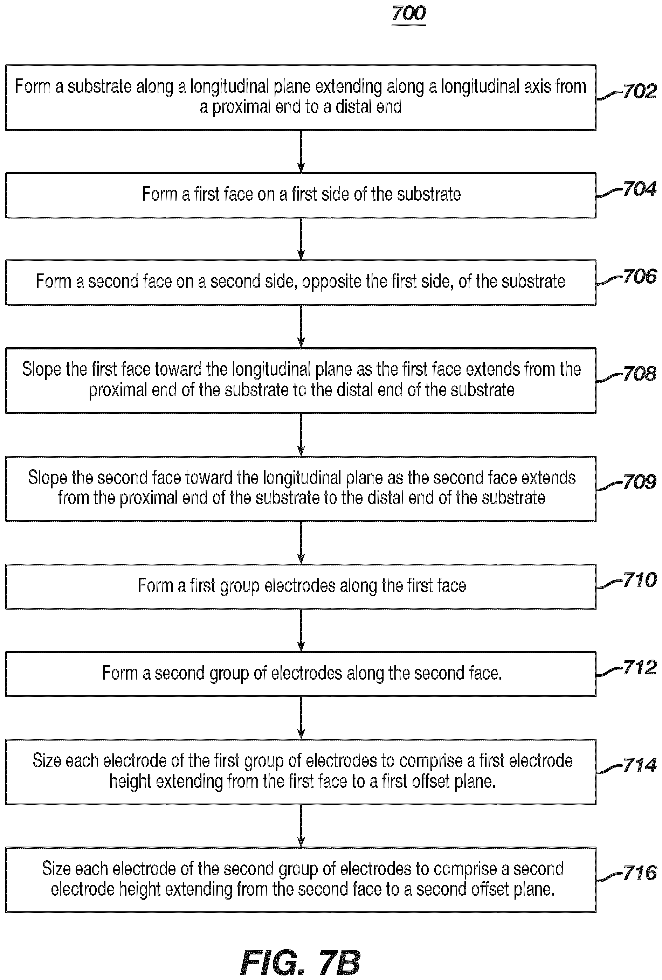

FIG. 7B shows the method further including sloping 709 the second face toward the longitudinal plane as the second face extends from the proximal end of the substrate to the distal end of the substrate. The method can further include sizing 714 each electrode of the first group of electrodes to include a first electrode height extending from the first face to a first offset plane. Method 700 can include sizing 716 each electrode of the second group of electrodes to include a second electrode height extending from the second face to a second offset plane.

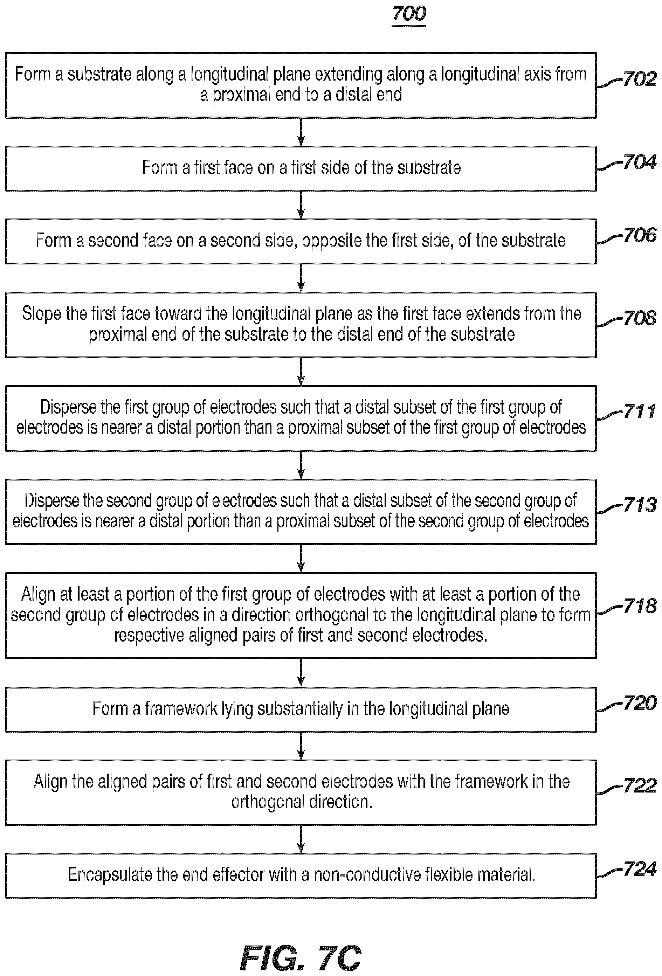

As shown in FIG. 7C, forming 710 the first group of electrodes can include dispersing 711 the first group of electrodes such that a distal subset of the first group of electrodes is nearer a distal portion than a proximal subset of the first group of electrodes, the distal subset of the first group of electrodes including approximately twice as many electrodes as the proximal subset of the first group of electrodes.

Method 700 can further include forming 712 the second group of electrodes. Forming 712 the second electrodes 712 can include dispersing 713 the second group of electrodes such that a distal subset of the second group of electrodes is nearer the distal portion than a proximal subset of the second group of electrodes, the distal subset of the second group of electrodes including approximately twice as many electrodes as the proximal subset of the second group of electrodes.

Additionally, the method 700 can include aligning 718 at least a portion of the first group of electrodes with at least a portion of the second group of electrodes in a direction orthogonal to the longitudinal plane to form respective aligned pairs of first and second electrodes. Method 700 can include forming 720 a framework lying substantially in the longitudinal plane and aligning 722 the aligned pairs of first and second electrodes with the framework in the orthogonal direction. Method 700 can further include encapsulating 724 the end effector with a non-conductive flexible material.

It should be appreciated that the method 700 just described can include more or fewer steps or features than those described. Furthermore, the steps or features can be performed in different orders and should not be construed as being required to be performed in any particular order.

The disclosed technology described herein can be further understood according to the following clauses:
Clause 1: An end effector for a catheter, the end effector comprising: a substrate extending along a longitudinal axis from a proximal portion of the end effector to a distal portion of the end effector and comprising: a longitudinal plane, the longitudinal axis running through the longitudinal plane; a first face disposed on a first side of the substrate and oblique to the longitudinal plane; and a second face, opposite the first face, disposed on a second side of the substrate; a first group of electrodes disposed on the first face; and a second group of electrodes disposed on the second face.
Clause 2: The end effector of clause 1, the second face oblique to the longitudinal plane.
Clause 3: The end effector of clause 1, further comprising: a first offset plane offset from the first face and parallel to the longitudinal plane; and a second offset plane offset from second face and parallel to the longitudinal plane.
Clause 4: The end effector of clause 3, the first group of electrodes extending outwardly from the first face to the first offset plane.
Clause 5: The end effector of clause 4, the second group of electrodes extending outwardly from the second face to the second offset plane.
Clause 6: The end effector of clause 3, the first group of electrodes each comprising an individual height from the first face to the first offset plane.
Clause 7: The end effector of clause 6, the second group of electrodes each comprising an individual height from the second face to the second offset plane.
Clause 8: The end effector of clause 3, the first offset plane parallel to the longitudinal plane and separated therefrom by a first distance.
Clause 9: The end effector of clause 8, the second offset plane parallel to the longitudinal plane and separated therefrom by a second distance.
Clause 10: The end effector of clause 9, wherein the second distance is equal to the first distance.
Clause 11: The end effector of clause 3, a distal end of the first face is separated from the first offset plane by a first distal distance, a proximal end of the first face is separated from the first offset plane by a first proximal distance, and the first distal distance does not equal the first proximal distance.
Clause 12: The end effector of clause 11, a distal end of the second face is separated from the second offset plane by a second distal distance, a proximal end of the second face is separated from the second offset plane by a second proximal distance, and the second distal distance does not equal the second proximal distance.
Clause 13: The end effector of clause 12, wherein the first proximal distance is equal to the second proximal distance, and wherein the first distal distance is equal to the second distal distance.
Clause 14: The end effector of any of clauses 1-13, the first group of electrodes being disposed on the first face such that a distal subset of the first group of electrodes is nearer the distal portion than a proximal subset of the first group of electrodes, the distal subset of the first group of electrodes comprising approximately twice as many electrodes as the proximal subset of the first group of electrodes.
Clause 15: The end effector of any of clauses 1-14, the second group of electrodes being disposed on the second face such that a distal subset of the second group of electrodes is nearer the distal portion than a proximal subset of the second group of electrodes, the distal subset of the second group of electrodes comprising approximately twice as many electrodes as the proximal subset of the second group of electrodes.
Clause 16: The end effector of any of clauses 1-15, at least a portion of the first group of electrodes being aligned with at least a portion of the second group of electrodes in a direction orthogonal to the longitudinal plane to form respective aligned pairs of first and second electrodes.
Clause 17: The end effector of any of clauses 1-16, further comprising a framework lying in the substrate and coplanar with the longitudinal plane.
Clause 18: The end effector of clause 17, the framework comprising a plurality of tines, the plurality of tines joined proximate a proximal end of the end effector and extending distally therefrom.
Clause 19: The end effector of clause 18, the plurality of tines comprising two outer tines and two inner tines, an outer tine and an inner tine disposed on either side of the longitudinal axis.
Clause 20: The end effector of clause 19, the two inner tines narrowing progressively in a distal direction.
Clause 21: The end effector of any of clauses 16-20, the aligned pairs of first and second electrodes aligned with the framework in the orthogonal direction.
Clause 22: The end effector of any of clauses 19-21, the substrate further defining a plurality of elongate slots, the elongate slots generally parallel to the longitudinal axis and disposed between each outer tine and inner tine.
Clause 23: The end effector of any of clauses 1-22, the substrate comprising a flexible circuit.
Clause 24: The end effector of any of clauses 1-16, the substrate comprising a framework.
Clause 25: The end effector of clause 24, the framework comprising a plurality of tines, the plurality of tines joined proximate a proximal end of the end effector and extending distally therefrom.
Clause 26: The end effector of clause 25, the plurality of tines comprising two outer tines and two inner tines, an outer tine and an inner tine disposed on either side of the longitudinal axis.
Clause 27: The end effector of clause 26, the two inner tines narrowing progressively in a distal direction.
Clause 28: The end effector of any of clauses 24-27, the framework comprising nitinol.
Clause 29: The end effector of any of clauses 1-28, the end effector being at least partially encapsulated by a non-conductive flexible material.
Clause 30: The end effector of any of clauses 1-29, the first group of electrodes comprising at least one first mapping electrode.
Clause 31: The end effector of any of clauses 1-30, the first group of electrodes comprising at least one first ablation electrode.
Clause 32: The end effector of any of clauses 1-31, the second group of electrodes comprising at least one second mapping electrode.
Clause 33: The end effector of any of clauses 1-32, the second group of electrodes comprising at least one second ablation electrode.
Clause 34: A nonplanar end effector for a mapping catheter, the end effector comprising: a sloped substrate extending along a longitudinal plane extending along a longitudinal axis from a proximal end of the end effector to a distal end of the end effector, the substrate thinning progressively in a direction orthogonal to the longitudinal plane; a first plurality of electrodes disposed on a first face of the sloped substrate; and a second plurality of electrodes disposed on a second face of the sloped substrate.
Clause 35: The end effector of clause 34, the substrate further defining a plurality of elongate slots, the elongate slots generally parallel to the longitudinal axis.
Clause 36: The end effector of any of clauses 34-35, the first plurality of electrodes comprising a respective plurality of first contact surfaces, the plurality of first contact surfaces being parallel to the first face.
Clause 37: The end effector of any of clauses 34-35, the first plurality of electrodes comprising a respective plurality of first contact surfaces, the plurality of first contact surfaces being parallel to the longitudinal plane.
Clause 38: The end effector of any of clauses 34-37, the second plurality of electrodes comprising a respective plurality of second contact surfaces, the plurality of second contact surfaces being parallel to the second face.
Clause 39: The end effector of any of clauses 34-37, the second plurality of electrodes comprising a respective plurality of second contact surfaces, the plurality of second contact surfaces being parallel to the longitudinal plane.
Clause 40: The end effector of any of clauses 34-39, the first plurality of electrodes being disposed on the first face such that a distal subset of the first plurality of electrodes is nearer the distal end than a proximal subset of the first plurality of electrodes, the distal subset of the first plurality of electrodes comprising approximately twice as many electrodes as the proximal subset of the first plurality of electrodes.
Clause 41: The end effector of any of clauses 34-40, the second plurality of electrodes being disposed on the second face such that a distal subset of the second plurality of electrodes is nearer the distal end than a proximal subset of the second plurality of electrodes, the distal subset of the second plurality of electrodes comprising approximately twice as many electrodes as the proximal subset of the second plurality of electrodes.
Clause 42: The end effector of any of clauses 34-41, wherein the sloped substrate is a flex circuit.
Clause 43: The end effector of any of clauses 34-42, the end effector further comprising: a framework affixed to the substrate by a non-conductive flexible encapsulating material.
Clause 44: The end effector of any of clauses 34-41, wherein the sloped substrate is a framework.
Clause 45: The end effector of any of clauses 43-44, the framework comprising a plurality of tines, the plurality of tines joined proximate the proximal end of the end effector and extending distally therefrom.
Clause 46: The end effector of clause 45, the plurality of tines comprising two outer tines and two inner tines, an outer tine and an inner tine disposed on either side of the longitudinal axis.
Clause 47: The end effector of clause 46, the two inner tines narrowing progressively in a distal direction.
Clause 48: The end effector of any of clauses 43-47, each of the first plurality of electrodes being aligned with a respective second electrode and the framework in the orthogonal direction.
Clause 49: The end effector of any of clauses 43-48, the framework comprising nitinol.
Clause 50: The end effector of any of clauses 34-48, the first plurality of electrodes comprising at least one first mapping electrode.
Clause 51: The end effector of any of clauses 34-50, the first plurality of electrodes comprising at least one first ablation electrode.
Clause 52: The end effector of any of clauses 34-51, the second plurality of electrodes comprising at least one second mapping electrode.
Clause 53: The end effector of any of clauses 34-52, the second plurality of electrodes comprising at least one second ablation electrode.
Clause 54: A system for performing a medical procedure, the system comprising: an elongate shaft extending along a longitudinal axis from a proximal end to a distal end; an end effector disposed on the distal end of the elongate shaft, the end effector comprising: a substrate extending along a longitudinal axis from a proximal portion of the end effector to a distal portion of the end effector and comprising: a first face disposed on a first side of a longitudinal plane and oblique thereto; a second face disposed on a second side of the longitudinal plane and oblique thereto; a first group of electrodes disposed on the first face; and a second group of electrodes disposed on the second face.
Clause 55: The system of clause 54, the system further comprising a pull wire extending through the elongate shaft and comprising a distal end coupled to the proximal portion of the end effector.
Clause 56: The system of clause 54, the second face oblique to the longitudinal plane.
Clause 57: The system of clause 54, further comprising: a first offset plane offset from the first face and parallel to the longitudinal plane; and a second offset plane offset from second face and parallel to the longitudinal plane.
Clause 58: The system of clause 57, the first group of electrodes extending outwardly from the first face to the first offset plane.
Clause 59: The system of clause 58, the second group of electrodes extending outwardly from the second face to the second offset plane.
Clause 60: The system of clause 57, the first group of electrodes each comprising an individual height from the first face to the first offset plane.
Clause 61: The system of clause 60, the second group of electrodes each comprising an individual height from the second face to the second offset plane.
Clause 62: The system of clause 57, the first offset plane parallel to the longitudinal plane and separated therefrom by a first distance.
Clause 63: The system of clause 62, the second offset plane parallel to the longitudinal plane and separated therefrom by a second distance.
Clause 64: The system of clause 63, wherein the second distance is equal to the first distance.
Clause 65: The system of clause 57, a distal end of the first face is separated from the first offset plane by a first distal distance, a proximal end of the first face is separated from the first offset plane by a first proximal distance, and the first distal distance does not equal the first proximal distance.
Clause 66: The system of clause 65, a distal end of the second face is separated from the second offset plane by a second distal distance, a proximal end of the second face is separated from the second offset plane by a second proximal distance, and the second distal distance does not equal the second proximal distance.
Clause 67: The system of clause 66, wherein the first proximal distance is equal to the second proximal distance, and wherein the first distal distance is equal to the second distal distance.
Clause 68: The system of any of clauses 54-67, the first group of electrodes being disposed on the first face such that a distal subset of the first group of electrodes is nearer the distal portion than a proximal subset of the first group of electrodes, the distal subset of the first group of electrodes comprising approximately twice as many electrodes as the proximal subset of the first group of electrodes.
Clause 69: The system of any of clauses 54-68, the second group of electrodes being disposed on the second face such that a distal subset of the second group of electrodes is nearer the distal portion than a proximal subset of the second group of electrodes, the distal subset of the second group of electrodes comprising approximately twice as many electrodes as the proximal subset of the second group of electrodes.
Clause 70: The system of any of clauses 54-69, at least a portion of the first group of electrodes being aligned with at least a portion of the second group of electrodes in a direction orthogonal to the longitudinal plane to form respective aligned pairs of first and second electrodes.
Clause 71: The system of any of clauses 54-70, further comprising a framework lying in the substrate and coplanar with the longitudinal plane.
Clause 72: The system of clause 71, the framework comprising a plurality of tines, the plurality of tines joined proximate a proximal end of the end effector and extending distally therefrom.
Clause 73: The system of clause 72, the plurality of tines comprising two outer tines and two inner tines, an outer tine and an inner tine disposed on either side of the longitudinal axis.
Clause 74: The system of clause 73, the two inner tines narrowing progressively in a distal direction.
Clause 75: The system of any of clauses 70-74, the aligned pairs of first and second electrodes aligned with the framework in the orthogonal direction.
Clause 76: The system of any of clauses 73-75, the substrate further defining a plurality of elongate slots, the elongate slots generally parallel to the longitudinal axis and disposed between the outer tine and the inner tine.
Clause 77: The system of any of clauses 54-76, the substrate comprising a flexible circuit.
Clause 78: The system of any of clauses 54-70, the substrate comprising a framework.
Clause 79: The system of clause 78, the framework comprising a plurality of tines, the plurality of tines joined proximate a proximal end of the end effector and extending distally therefrom.
Clause 80: The system of clause 79, the plurality of tines comprising two outer tines and two inner tines, an outer tine and an inner tine disposed on either side of the longitudinal axis.
Clause 81: The system of clause 80, the two inner tines narrowing progressively in a distal direction.
Clause 82: The system of any of clauses 78-81, the framework comprising nitinol.
Clause 83: The system of any of clauses 54-82, the end effector being at least partially encapsulated by a non-conductive flexible material.
Clause 84: The system of any of clauses 54-83, the first group of electrodes comprising at least one first mapping electrode.
Clause 85: The system of any of clauses 54-84, the first group of electrodes comprising at least one first ablation electrode.
Clause 86: The system of any of clauses 54-85, the second group of electrodes comprising at least one second mapping electrode.
Clause 87: The system of any of clauses 54-86, the second group of electrodes comprising at least one second ablation electrode.
Clause 88: A method of providing a stiffened flexible end effector, comprising: forming a substrate along a longitudinal plane extending along a longitudinal axis from a proximal end to a distal end; forming a first face on a first side of the substrate; forming a second face on a second side, opposite the first side, of the substrate; sloping the first face toward the longitudinal plane as the first face extends from the proximal end of the substrate to the distal end of the substrate; forming a first group electrodes along the first face; and forming a second group of electrodes along the second face.
Clause 89: The method of clause 88, further comprising: sloping the second face toward the longitudinal plane as the second face extends from the proximal end of the substrate to the distal end of the substrate.
Clause 90: The method of any of clauses 88-89, further comprising: sizing each electrode of the first group of electrodes to comprise a first electrode height extending from the first face to a first offset plane.
Clause 91: The method of any of clauses 88-90, further comprising:
   sizing each electrode of the second group of electrodes to comprise a second electrode height extending from the second face to a second offset plane.
Clause 92: The method of clause 91, the first offset plane parallel to and separate from the longitudinal plane, and the second offset plane parallel to and separate from the longitudinal plane.
Clause 93: The method of clause 90, the first offset plane oblique to and separate from the longitudinal plane, and the second offset plane oblique to and separate from the longitudinal plane.
Clause 94: The method of any of clauses 88-93, wherein forming the first group of electrodes comprises dispersing the first group of electrodes such that a distal subset of the first group of electrodes is nearer a distal portion than a proximal subset of the first group of electrodes, the distal subset of the first group of electrodes comprising approximately twice as many electrodes as the proximal subset of the first group of electrodes.
Clause 95: The method of any of clauses 88-94, wherein forming the second group of electrodes comprises dispersing the second group of electrodes such that a distal subset of the second group of electrodes is nearer the distal portion than a proximal subset of the second group of electrodes, the distal subset of the second group of electrodes comprising approximately twice as many electrodes as the proximal subset of the second group of electrodes.
Clause 96: The method of clause 95, further comprising: aligning, at least a portion of the first group of electrodes with at least a portion of the second group of electrodes in a direction orthogonal to the longitudinal plane to form respective aligned pairs of first and second electrodes.
Clause 97: The method of clause 96, further comprising: forming a framework lying substantially in the longitudinal plane.
Clause 98: The method of clause 97, further comprising: aligning the aligned pairs of first and second electrodes with the framework in the orthogonal direction.
Clause 99: The method of clause 98, further comprising: encapsulating the end effector with a non-conductive flexible material.
Clause 100: The method of any of clauses 88-99, the first group of electrodes comprising at least one first mapping electrode.
Clause 101: The method of any of clauses 88-100, the first group of electrodes comprising at least one first ablation electrode.
Clause 102: The method of any of clauses 88-101, the second group of electrodes comprising at least one second mapping electrode.
Clause 103: The method of any of clauses 88-102, the second group of electrodes comprising at least one second ablation electrode.

## Claims

1. An end effector for a catheter, the end effector comprising:
a substrate extending along a longitudinal axis from a proximal portion of the end effector to a distal portion of the end effector and comprising:
a longitudinal plane, the longitudinal axis running through the longitudinal plane;
a first face disposed on a first side of the substrate and oblique to the longitudinal plane;
a second face, opposite the first face, disposed on a second side of the substrate;
a first group of electrodes disposed on the first face; and
a second group of electrodes disposed on the second face.

2. The end effector of claim 1, the second face oblique to the longitudinal plane.

3. The end effector of claim 1, further comprising:
a first offset plane offset from the first face and parallel to the longitudinal plane; and
a second offset plane offset from second face and parallel to the longitudinal plane.

4. The end effector of claim 3, the first group of electrodes extending outwardly from the first face to the first offset plane, optionally the second group of electrodes extending outwardly from the second face to the second offset plane.

5. The end effector of claim 3, the first group of electrodes each comprising an individual height from the first face to the first offset plane, optionally the second group of electrodes each comprising an individual height from the second face to the second offset plane.

6. The end effector of any preceding claim, the first group of electrodes being disposed on the first face such that a distal subset of the first group of electrodes is nearer the distal portion than a proximal subset of the first group of electrodes, the distal subset of the first group of electrodes comprising approximately twice as many electrodes as the proximal subset of the first group of electrodes.

7. The end effector of any preceding claim, the second group of electrodes being disposed on the second face such that a distal subset of the second group of electrodes is nearer the distal portion than a proximal subset of the second group of electrodes, the distal subset of the second group of electrodes comprising approximately twice as many electrodes as the proximal subset of the second group of electrodes.

8. The end effector of any preceding claim, at least a portion of the first group of electrodes being aligned with at least a portion of the second group of electrodes in a direction orthogonal to the longitudinal plane to form respective aligned pairs of first and second electrodes.

9. The end effector of any preceding claim, further comprising a framework lying in the substrate and coplanar with the longitudinal plane.

10. The end effector of claim 3, the first offset plane parallel to the longitudinal plane and separated therefrom by a first distance, optionally the second offset plane parallel to the longitudinal plane and separated therefrom by a second distance.

11. The end effector of claim 3, a distal end of the first face is separated from the first offset plane by a first distal distance,
a proximal end of the first face is separated from the first offset plane by a first proximal distance, and
the first distal distance does not equal the first proximal distance,
optionally a distal end of the second face is separated from the second offset plane by a second distal distance,
a proximal end of the second face is separated from the second offset plane by a second proximal distance, and
the second distal distance does not equal the second proximal distance,
further optionally wherein the first proximal distance is equal to the second proximal distance, and wherein the first distal distance is equal to the second distal distance.

12. A nonplanar end effector for a mapping catheter, the nonplanar end effector comprising: a sloped substrate extending along a longitudinal plane extending along a longitudinal axis from a proximal end of the nonplanar end effector to a distal end of the nonplanar end effector, the sloped substrate thinning progressively in a direction orthogonal to the longitudinal plane; a first plurality of electrodes disposed on a first face of the sloped substrate; and a second plurality of electrodes disposed on a second face of the sloped substrate.

13. The nonplanar end effector of claim 12, the first plurality of electrodes comprising a respective plurality of first contact surfaces, the plurality of first contact surfaces being parallel to the first face.

14. The nonplanar end effector of claim 12, the first plurality of electrodes comprising a respective plurality of first contact surfaces, the plurality of first contact surfaces being parallel to the longitudinal plane.

15. A system for performing a medical procedure, the system comprising: an elongate shaft extending along a longitudinal axis from a proximal end to a distal end; an end effector disposed on the distal end of the elongate shaft, the end effector comprising: a substrate extending along a longitudinal axis from a proximal portion of the end effector to a distal portion of the end effector and comprising: a first face disposed on a first side of a longitudinal plane and oblique thereto; a second face disposed on a second side of the longitudinal plane and oblique thereto; a first group of electrodes disposed on the first face; and a second group of electrodes disposed on the second face.
